# EUROPÄISCHE PATENTSCHRIFT

(11) **EP 2 291 361 B1**
(45) Veröffentlichungstag und Bekanntmachung des Hinweises auf die Patenterteilung: **03.04.2013**
(21) Anmeldenummer: 09753756.7
(22) Anmeldetag: 22.04.2009
(51) Int. Cl.: C07D 251/38

(54) **VERFAHREN ZUR HERSTELLUNG DES TRINATRIUMSALZES DES 2, 4, 6-TRIMERCAPTO-S-TRIAZINS**
METHOD FOR PRODUCING THE TRISODIUM SALT OF 2,4,6-TRIMERCAPTO-S-TRIAZINE
PROCÉDÉ DE PRODUCTION DU SEL TRISODIQUE DE LA TRIMERCAPTO-S-TRIAZINE 2,4,6

(30) Priorität: 29.05.2008 DE 102008002073
(43) Veröffentlichungstag der Anmeldung: 09.03.2011
(73) Patentinhaber: Evonik Degussa GmbH, 45128 Essen (DE)
(72) Erfinder: GRIMMER, Jörg, 63500 Seligenstadt (DE); PELDSZUS, Rüdiger, 63594 Hasselroth (DE); VERELST, Peter, B-2070 Burcht (BE); VANDERVEE, Roger, B-3680 Neeroeteren (BE)
(86) Internationale Anmeldenummer: PCT/EP2009/054807
(87) Internationale Veröffentlichungsnummer: WO 2009/144101

(56) Entgegenhaltungen:
- EP-A- 0 413 980
- US-A- 5 563 267

## Beschreibung

Die vorliegende Erfindung beschreibt ein Verfahren zur Herstellung des Trinatriumsalzes des 2,4,6-Trimercapto-s-triazins (TMT-Na₃).

Die Herstellung des Trinatriumsalzes des 2,4,6-Trimercapto-s-triazins (TMT-Na₃ x 9 H₂O) wird in der DE 37 29 029 beschrieben. Zunächst wird Cyanurchlorid im wässrigen Medium bei Temperaturen von 20 bis 70°C mit NaSH, Na₂S oder einem NaSH/Na₂S-Gemisch umgesetzt, wobei ein pH-Wert > 7 eingestellt wird. Im Anschluss an die Reaktion wird das erhaltene Reaktionsgemisch auf 0 bis 20 °C abgekühlt und das auskristallisierte Trinatriumsalz (TMT-Na₃) abfiltriert. Dieses Filtrat wird mittels Salzsäure auf einen pH-Wert von 2 bis 4 eingestellt, das nun ausfallende 2,4,6-Trimercapto-s-triazin (TMT-H₃) wird abgetrennt und ohne Trocknung in eine Natriumhydroxidlösung eingetragen. Es entsteht eine wässrige 30 bis 40 Gew.-%ige Lösung an dem Trinatriumsalz (TMT-Na₃), die anschließend auf 0 bis 20°C abgekühlt wird, so dass nun das Trinatriumsalz (TMT-Na₃) auskristallisiert, welches abgetrennt werden kann. Das nun anfallende Filtrat wird dem Reaktor, in dem die Umsetzung des Cyanurchlorids stattfindet, zugeführt.

Die EP 0 413 980 A1 beschreibt ein ähnliches Herstellungsverfahren - jedoch zur Herstellung des Dinatriumsalz des 2,4,6-Trimercapto-s-triazins (TMT-Na₂ x 6 H₂O), wobei für die Aufarbeitung auch hier das Filtrat und das Waschwasser nach der Abtrennung des auskristallisierten Trinatriumsalzes (TMT-Na₃) mit Salzsäure angesäuert und das ausgefallene 2,4,6-Trimercapto-s-triazin (TMT-H₃) abgetrennt wird. Dieses abgetrennte 2,4,6-Trimercapto-s-triazin (TMT-H₃) als auch das abgetrennte Trinatriumsalzes (TMT-Na₃) werden nun in Wasser eingerührt. Diese wässrige Lösung wird eingeengt und auf 10 °C abgekühlt, wobei ein Feststoff auskristallisiert, der anschließend abgetrennt wird. Nach dem vorsichtigen Trocknen erhält man das Dinatriumsalz des 2,4,6-Trimercapto-s-triazins (TMT-Na₂).

Ebenfalls ein Verfahren zur Herstellung des Trinatriumsalzes des 2,4,6-Trimercapto-s-triazins (TMT-Na₃) beschreiben Liu et al. in Wuhan Huagong Xueyuan Xuebao (2005), 27(4), 8-10. Im Gegensatz zu den vorangegangen Verfahren wird hier das Reaktionsgemisch nicht abgekühlt, sondern die ungelösten Stoffe werden abfiltriert, es wird Salzsäure zugegeben, der sich bildende Niederschlag (TMT-H₃) wird ebenfalls abgetrennt. Anschließend erfolgt eine Umkristallisation des 2,4,6-Trimercapto-s-triazin (TMT-H₃) in Methanol. Dieses 2,4,6-Trimercapto-s-triazin (TMT-H₃) wird nun in eine wässrige Natriumhydroxidlösung gegeben, die nicht löslichen Bestandteile werden abfiltriert. Der pH-Wert wird auf 12 bis 12,5 eingestellt, man erhält eine 15%ige Lösung des Trinatriumsalzes des 2,4,6-Trimercapto-s-triazins (TMT-Na₃).

In der koreanischen Patentschrift KR 10-0695193 wird ein Verfahren zur Herstellung von Alkalimetallsalzen von 2,4,6-Trimercapto-s-triazin beschrieben, hierbei wird die erhaltene Reaktionslösung mittels Zugabe von Salzsäure auf einen pH-Wert von 6 oder niedriger eingestellt, so dass 2,4,6-Trimercapto-s-triazin (TMT-H₃) sich absetzt. Dieses wird abgetrennt, in Wasser dispergiert und mit Natronlauge versetzt, so dass sich das das Trinatriumsalz des 2,4,6-Trimercapto-s-triazins (TMT-Na₃) bildet. Die Lösung wird eingeengt und auf 0 bis 20 °C abgekühlt, das Trinatriumsalz (TMT-Na₃) wird abgetrennt und das Filtrat wird in das Verfahren zurückgeführt.

Die US 5,563,267 beschreibt ebenfalls ein Verfahren zur Herstellung von Trialkalimetallsalzen des Trimercapto-s-triazins in einem Aceton-Wasser-Gemisch. Das Reaktionsgemisch wird auf Raumtemperatur abgekühlt und mit einer Säure versetzt. Der entstehende Niederschlag wird abgetrennt und in eine basische Lösung gegeben. Der sich nun bildende Niederschlag (Trialkalimetallsalz des Trimercapto-s-triazins) wird abgetrennt.

Ein Verfahren zur Herstellung von 2,4,6-Trimercapto-s-triazin (TMT-H₃) beschreibt die DE 10 2005 036 693 A1, hierbei wird das 2,4,6-Trimercapto-s-triazin (TMT-H₃) bei einem pH-Wert von 1,5 bis 2,5 gefällt.

Es war die Aufgabe der vorliegenden Erfindung, ein Verfahren zur Herstellung des Trinatriumsalzes von 2,4,6-Trimercapto-s-triazin bereitzustellen, das es ermöglicht den Verbrauch an Säuren und Basen bei vergleichbarer Ausbeute - wie beim Stand der Technik - zu reduzieren.

Überraschenderweise wurde ein Verfahren zur Herstellung des Trinatriumsalzes von 2,4,6-Trimercapto-s-triazin (TMT-Na₃) gefunden, das sich dadurch auszeichnet, dass die Mutterlauge nach der Abtrennung des Trinatriumsalzes von 2,4,6-Trimercapto-s-triazins (TMT-Na₃) auf einen pH-Wert von 5,5 bis 8 eingestellt wird. Im Gegensatz dazu wird bei Verfahren gemäß dem Stand der Technik die Mutterlauge auf einen pH-Wert von 2 bis 4 eingestellt, wie beispielsweise in DE 37 29 029 A1 beschrieben. Durch die Absenkung des pH-Wertes von ≥ 12 auf einen pH-Wert von 5,5 bis 8 ist deutlich weniger Säure notwendig, als bei einem Verfahren, das direkt nach der Abtrennung des Trinatriumsalzes von 2,4,6-Trimercapto-s-triazin (TMT-Na₃) einen pH-Wert von kleiner 4 benötigt. Bei einem pH-Wert von 5,5 bis 8 kann ebenfalls die in der Mutterlauge noch verbliebenen 2,4,6-Trimercapto-s-triazin-Verbindungen ausgefällt werden, jedoch als Mononatriumsalz (TMT-Na₁) anstatt in Form des 2,4,6-Trimercapto-s-triazins (TMT-H₃). Bei dem erfindungsgemäßen Verfahren wird das aus der Mutterlauge ausgefällte und abgetrennte Mononatriumsalz (TMT-Na₁) durch pH-Werterhöhung wieder in Lösung gebracht. Vorzugsweise handelt es sich hierbei um denselben pH-Wert wie er auch bei der Umsetzung des Cyanurchlorids eingestellt wird. Auf diese Weise kann diese Lösung dem Reaktor nach der Reaktion aber vor der Ausfällung des Trinatriumsalzes (TMT-Na₃) zurückgeführt werden. Bei Verfahren gemäß dem Stand der Technik wird das 2,4,6-Trimercapto-s-triazin (TMT-H₃) in Natronlauge gegeben, um wiederum das Trinatriumsalz (TMT-Na₃) ausfällen zu können. Da das Trinatriumsalz (TMT-Na₃) in der Regel bei einem pH-Wert ≥ 12 ausfällt, ist nun eine pH-Werterhöhung von 2 bis 4 auf ≥ 12 notwendig. Somit sind in den Verfahren gemäß dem Stand der Technik erhebliche Mengen an Säuren und Basen nötig. Hingegen sind bei dem erfindungsgemäßen Verfahren die pH-Wertänderungen kleiner und somit wird weniger Säure bzw. Base benötigt. Überraschend war es ferner, dass neben dem Einsparen an Säure und Base auch die Gesamtausbeute an dem Trinatriumsalz (TMT-Na₃) mit der Gesamtausbeute von Verfahren gemäß dem Stand der Technik vergleichbar ist.

Weiterer Vorteil des erfindungsgemäßen Verfahrens ist die Möglichkeit den bei der Umsetzung des Cyanurchlorids entstehenden Schwefelwasserstoff zu isolieren, mit Natronlauge umzusetzen und der Reaktion zurückzuführen. Auf diese Weise kann die Wirtschaftlichkeit und die Umweltverträglichkeit des erfindungsgemäßen Verfahren noch weiter bessert werden.

Gegenstand der Erfindung ist somit ein Verfahren zur Herstellung des Trinatriumsalzes des 2,4,6-Trimercapto-s-triazins (TMT-Na₃), wobei Cyanurchlorid in einem wässrigen Medium bei einer Temperatur von 20 bis 70°C mit NaSH, Na₂S oder einer Mischung dieser beiden Verbindungen bei einem pH-Wert von 7 bis 11 umgesetzt wird und anschließend bei einem pH-Wert von ≥ 12 und einer Temperatur von 0 bis 20 °C das Trinatriumsalz des 2,4,6-Trimercapto-s-triazins (TMT-Na₃) ausgefällt und abgetrennt wird, das dadurch gekennzeichnet ist, dass die Mutterlauge nach dem Abtrennschritt des Trinatriumsalzes des 2,4,6-Trimercapto-s-triazins (TMT-Na₃) auf einen pH-Wert von 6 bis 8 eingestellt wird, wobei das Mononatriumsalz des 2,4,6-Trimercapto-s-triazins (TMT-Na₁) ausfällt.

Bei der Umsetzung des Cyanurchlorids in dem erfindungsgemäßen Verfahrens werden als schwefelhaltige Komponente vorzugsweise NaSH und/oder Na₂S eingesetzt. Es kann auch eine Mischung aus NaSH und Na₂S eingesetzt werden, bevorzugt mit einem molaren Verhältnis von Na₂S zu NaSH von 1:1 bis 1:4, insbesondere von 1:2 bis 1:4. Als weitere schwefelhaltige Komponente kann auch eine wässrige Lösung an NaSH, Na₂S und Natronlauge, wie sie bei der Gaswäsche von Schwefelwasserstoff mittels Natronlauge entsteht, eingesetzt werden. Vorzugsweise wird sowohl NaSH und/oder Na₂S als auch eine wässrige Lösung an NaSH, Na₂S und Natronlauge eingesetzt. Das molare Verhältnis von der Gesamtmenge an Schwefel zum Cyanurchlorid beträgt bei der Reaktion vorzugsweise mindestens 3 : 1, bevorzugt jedoch 3,5 : 1 bis 9 : 1 und besonders bevorzugt von 4 : 1 bis 8 : 1. Die Umsetzung des Cyanurchlorids erfolgt in dem erfindungsgemäßen Verfahren vorzugsweise bei einem pH-Wert von 9 bis 11, bevorzugt von 10 bis 11. Die Temperatur beträgt hierbei vorzugsweise 40 bis 60°C, bevorzugt von 45 bis 55°C und besonders bevorzugt 50°C. Im Anschluss an die Reaktion bzw. Zugabe der Edukte kann in dem erfindungsgemäßen Verfahren eine Nachreaktion bei derselben Temperatur als auch bei demselben pH-Wert wie die Hauptreaktion durchgeführt werden.

Damit das Trinatriumsalz des 2,4,6-Trimercapto-s-triazins (TMT-Na₃) nahezu vollständig ausgefällt werden kann, wird nach der Reaktion bzw. nach der Nachreaktion des erfindungsgemäßen Verfahrens der pH-Wert der Reaktionslösung vorzugsweise auf ≥ 12, bevorzugt auf einen pH-Wert von 12 bis 13,5 eingestellt. Ferner ist es empfehlenswert die Reaktionslösung auf eine Temperatur von 5 bis 30°C, bevorzugt auf 10 bis 20°C abzukühlen. Das auf diese Weise ausgefällte Trinatriumsalz des 2,4,6-Trimercapto-s-triazins (TMT-Na₃) kann nun mittels eines Trennverfahrens abgetrennt werden, vorzugsweise erfolgt die Abtrennung des Trinatriumsalzes in dem erfindungsgemäßen Verfahrens mittels eines mechanischen Trennverfahrens, wie beispielsweise Filtration, Zentrifugieren oder Dekantieren.

Das Filtrat bzw. nach der Abtrennung des Trinatriumsalzes (TMT-Na₃) vorliegende wässrige Phase wird im Rahmen dieser Erfindung als Mutterlauge bezeichnet. Vorzugsweise wird diese Mutterlauge in dem erfindungsgemäßen Verfahren auf einen pH-Wert von 6 bis 8, bevorzugt auf einen pH-Wert von 6,5 bis 7,5 und besonders bevorzugt auf einen pH-Wert von 7 eingestellt. Der pH-Wert kann mittels einer anorganischen Säure eingestellt werden, bevorzugt erfolgt dies mittels Salzsäure und besonders bevorzugt mittels einer 10 bis 25 %igen und ganz besonders bevorzugt mittels einer 16 bis 22 %igen Salzsäure. Bei diesem pH-Wert kann das Mononatriumsalz des 2,4,6-Trimercapto-s-triazins (TMT-Na₁) ausgefällt werden, das ähnlich wie das 2,4,6-Trimercapto-s-triazin (TMT-H₃) eine geringe Löslichkeit in Wasser aufweist. Vorzugsweise erfolgt diese bei einer Temperatur von 10 bis 45°C, bevorzugt bei einer Temperatur von 30 bis 45°C.

Das ausgefällte Mononatriumsalz des 2,4,6-Trimercapto-s-triazins (TMT-Na₁) kann anschließend in dem erfindungsgemäßen Verfahren mittels eines Trennverfahrens, vorzugsweise mittels eines mechanischen Trennverfahrens, wie beispielsweise Filtration, Zentrifugieren oder Dekantieren, von der Mutterlauge abgetrennt werden.

Das abgetrennte Mononatriumsalz des 2,4,6-Trimercapto-s-triazins (TMT-Na₁) kann nun in dem erfindungsgemäßen Verfahrens bei einem pH-Wert von 9 bis 11, bevorzugt bei einem pH-Wert von 10 bis 11 in einem wässrigen Medium in Lösung gebracht werden. Vorzugsweise wird hierfür Natronlauge, bevorzugt eine 5 bis 60%ige, besonders bevorzugt eine 30 bis 55%ige Natronlauge eingesetzt. Als Temperatur kann eine Temperatur von 20 bis 50°C eingestellt werden. Bevorzugt wird das Mononatriumsalz des 2,4,6-Trimercapto-s-triazins (TMT-Na₁) mittels Natronlauge bei einem pH-Wert von 10 bis 11 und einer Temperatur von 20 bis 50°C in Lösung gebracht, wobei sich das Dinatriumsalz des 2,4,6-Trimercapto-s-triazins (TMT-Na₂) bildet.

Diese wässrige Lösung des Dinatriumsalzes des 2,4,6-Trimercapto-s-triazins (TMT-Na₂) kann nun in den Reaktor zurückgeführt werden, bevorzugt erfolgt dies nach der abgeschlossenen Reaktion und Nachreaktion des Cyanurchlorids, bevorzugt jedoch vor der Ausfällung des Trinatriumsalzes (TMT-Na₃).

Der pH-Wert des Filtrats aus der Abtrennung des Mononatriumsalzes des 2,4,6-Trimercapto-s-triazins (TMT-Na₁) kann nun noch weiter abgesenkt werden, vorzugsweise auf einen pH-Wert von 0 bis 4 und bevorzugt auf einen pH-Wert von 1,5 bis 2,5. Bei diesem pH-Wert kann nun das 2,4,6-Trimercapto-s-triazin (TMT-H₃) ausgefällt und mittels eines Trennverfahrens abgetrennt werden. Dieses ausgefällte 2,4,6-Trimercapto-s-triazin (TMT-H₃) kann in den Aufarbeitungsprozess der Mutterlauge zurückgeführt werden, vorzugsweise wird dieses der Mutterlauge vor dem Ausfällen des Mononatriumsalzes des 2,4,6-Trimercapto-s-triazins (TMT-Na₁) zugeführt.

Durch die Umsetzung des Cyanurchlorids mit NaSH, Na₂S oder einer Mischung dieser beiden Verbindungen wird Schwefelwasserstoff freigesetzt bzw. liegt in der wässrigen Lösung vor. Aus umweltschutztechnischen Gründen als auch um die Reinheit des Zielproduktes zu erhöhen, wird der entstandene Schwefelwasserstoff vorzugsweise aufgefangen und durch ein oder mehrere Gaswäscher mit Natronlauge geleitet. Diese mit Schwefelwasserstoff umgesetzte Natronlauge kann nun dem Reaktor wieder als Edukt zurückgeführt werden. Vorteilhaft ist es, der Mutterlauge vor dem Ausfällen des Mononatriumsalzes des 2,4,6-Trimercapto-s-triazins (TMT-Na₁) ein Unterdruck von < 1000 mbar angelegt wird, bevorzugt wird ein Druck von 150 bis 250 mbar angelegt. Der Druck und die Temperatur sollte so gewählt werden, dass möglichst wenig Wasser in die Gasphase übergeleitet wird, daher wird die Mutterlauge vorzugsweise bei einer Temperatur von 10 bis 55°C, bevorzugt von 30 bis 45°C entgast. Die entweichenden Gase werden nun vorzugsweise durch ein oder mehrere Gaswäscher mit Natronlauge geleitet. Die durch die Umsetzung von Schwefelwasserstoff und Natronlauge entstehende wässrige Lösung an NaSH, Na₂S und Natronlauge kann nun in den Reaktor, der für die Umsetzung des Cyanurchlorids mit NaSH und/oder Na₂S dient, zurückgeführt werden.

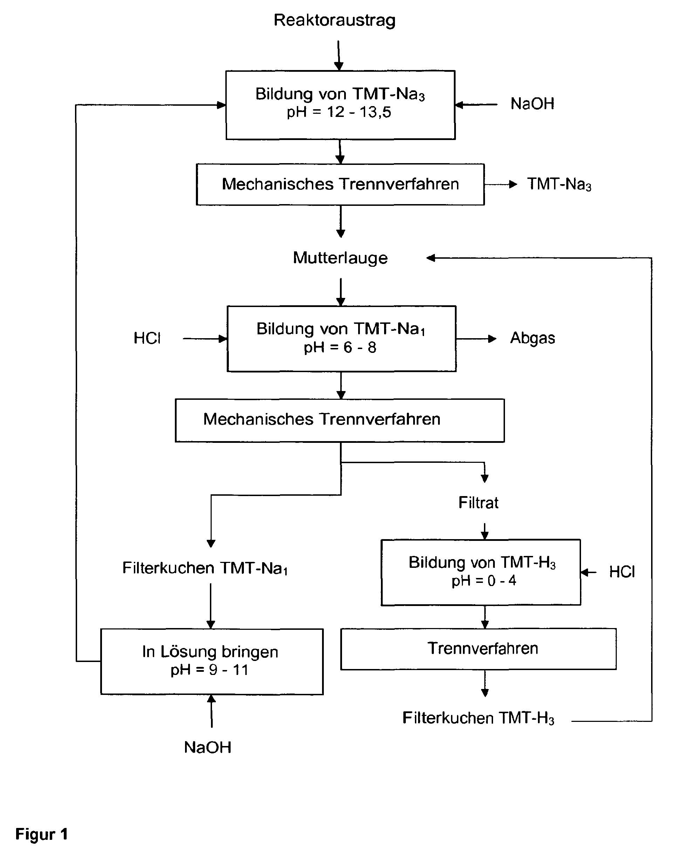

Figur 1 zeigt ein Vertahrensfließbild, das die bevorzugten Verfahrensschritte und Stoffströme des erfindungsgemäßen Verfahrens darstellt.

Die nachfolgenden Beispiele sollen das erfindungsgemäße Verfahren näher erläutern, ohne dass die Erfindung auf diese Ausführungsform beschränkt sein soll.

### Beispiel 1:

In einem Reaktor werden 2,1 l einer 40 Gew.-%igen NaSH vorgelegt und 1,5 l einer 50 Gew.-%igen Natronlauge und eine wässrige Cyanurchlorid-Suspension (2,7 kg Cyanurchlorid in 4,5 l Wasser) werden bei einem pH-Wert von 10,5 und einer Temperatur von 50°C zudosiert. Nach der Zudosierung lässt man die Reaktionsmischung noch weitere 30 Minuten nachreagieren. Mittels einer 50 Gew.-%igen Natronlauge wird der pH-Wert auf 12,5 - 13 eingestellt. Man lässt die Reaktionsmischung auf 20°C abkühlen. Das sich nun bildende TMT-Na₃ wird nun mittels Zentrifuge von der Mutterlauge abgetrennt.

### Beispiel 2:

941,2 g der in Beispiel 1 hergestellten Mutterlauge werden mittels 227,6 g einer 16,22 Gew.-%igen Salzsäure auf pH = 2 eingestellt. Bei einem Druck von 200 mbar und einer Temperatur von 40°C wird die Mutterlauge entgast, es entweicht ein Gemisch aus Schwefelwasserstoff und Wasser. Gleichzeitig scheidet sich TMT-H3 ab, welches man absetzen lässt und das Filtrat anschließend abfiltriert. Dieser Filterkuchen (48,8 g) wird mit 63,0 g einer 19,75 Gew.-%igen Natronlauge und 71,8 g Wasser versetzt, so dass man einen pH-Wert von 12,5 erhält. Bei einer Temperatur von 40 - 45°C scheidet sich nun das TMT-Na₃ ab, welches wiederum mittels Filtrieren bei einer Temperatur von 20°C von dem Filtrat getrennt wird.

Der Verbrauch an HCl beträgt 1,025 mol und an NaOH 0,311 mol.

### Beispiel 3:

941,7 g der in Beispiel 1 hergestellten Mutterlauge werden mittels 164,4 g einer 16,22 Gew.-% igen Salzsäure auf pH = 7 eingestellt. Bei einem Druck von 200 mbar und einer Temperatur von 40°C wird die Mutterlauge entgast, es entweicht ein Gemisch aus Schwefelwasserstoff und Wasser. Gleichzeitig scheidet sich TMT-Na₁ ab, welches man absetzen lässt und das Filtrat anschließend abfiltriert. Das Filtrat wird nun mittels 47,7 g einer 16,22 Gew.-%igen Salzsäure auf pH = 2 gebracht. Bei einer Temperatur von 40°C scheidet sich nun TMT-H₃ ab, welches mittels Absetzen und Filtrieren abgetrennt wird. Der Filterkuchen mit TMT-Na₁ (40,5 g) und der Filterkuchen mit TMT-H₃ (10,7 g) werden vereinigt und mit 46,7 g einer 19,75 Gew.-%igen Natronlauge und 48,8 g Wasser versetzt, so dass man einen pH-Wert von 12,5 erhält. Bei einer Temperatur von 40 - 45°C scheidet sich nun das TMT-Na₃ ab, welches wiederum mittels Filtrieren bei einer Temperatur von 20°C von dem Filtrat getrennt wird.

Der Verbrauch an HCl beträgt (0,741 + 0,215) mol = 0,956 mol und an NaOH 0,230 mol.

Die beiden Beispiele 2 und 3 zeigen, dass der Verbrauch an Salzsäure und Natronlauge und damit die in der Anlage anfallende Kochsalzfracht in Beispiel 3 geringer ist.

### Beispiel 4:

Die in Beispiel 1 hergestellten Mutterlauge wird mittels einer 12 Gew.-%igen Salzsäure auf pH = 7 eingestellt. Bei einem Druck von 200 mbar und einer Temperatur von 45°C wird die Mutterlauge entgast, es entweicht ein Gemisch aus Schwefelwasserstoff und Wasser. Gleichzeitig scheidet sich TMT-Na₁ ab, welches man absetzen lässt und das Filtrat anschließend abdekantiert.

Der Filterkuchen mit TMT-Na₁ wird mit einer 50 Gew.-%igen Natronlauge versetzt und die erhaltene Lösung auf einen pH-Wert von 10,5 eingestellt. Die erhaltene Lösung wird dem Reaktorausgang vor Fällung des TMT-Na₃ zugeführt.

Das Filtrat aus der Abtrennung des TMT-Na₁ wird mittels einer 12 Gew.-%igen Salzsäure auf pH = 2 gebracht. Es scheidet sich nun TMT-H₃ ab, welches mittels Absetzen und Dekantieren abgetrennt und der Mutterlauge aus Beispiel 1 zugefügt wird.

Auf diese Weise lässt sich die Gesamtausbeute auf 92,9 % bezogen auf das eingesetzte Cyanurchlorid steigern.

## Patentansprüche

1. Verfahren zur Herstellung des Trinatriumsalzes des 2,4,6-Trimercapto-s-triazins (TMT-Na₃), wobei Cyanurchlorid in einem wässrigen Medium bei einer Temperatur von 20 bis 70°C mit NaSH, Na₂S oder einer Mischung dieser beiden Verbindungen bei einem pH-Wert von 7 bis 11 umgesetzt wird und anschließend bei einem pH-Wert von ≥ 12 und einer Temperatur von 0 bis 20 °C das Trinatriumsalz des 2,4,6-Trimercapto-s-triazins (TMT-Na₃) ausgefällt und abgetrennt wird,
**dadurch gekennzeichnet,**
**dass** die Mutterlauge nach dem Abtrennschritt des Trinatriumsalzes des 2,4,6-Trimercapto-s-triazins (TMT-Na₃) auf einen pH-Wert von 6 bis 8 eingestellt wird, wobei das Mononatriumsalz des 2,4,6-Trimercapto-s-triazins (TMT-Na₁) ausfällt.

2. Verfahren gemäß Anspruch 1,
**dadurch gekennzeichnet,**
**dass** die Mutterlauge auf eine Temperatur von 10 bis 45°C eingestellt wird.

3. Verfahren gemäß Anspruch 1 oder 2,
**dadurch gekennzeichnet,**
**dass** das ausgefällte Mononatriumsalz des 2,4,6-Trimercapto-s-triazins (TMT-Na₁) aus der Mutterlauge mittels eines mechanischen Trennverfahrens abgetrennt wird.

4. Verfahren gemäß Anspruch 3,
**dadurch gekennzeichnet,**
**dass** der pH-Wert des Filtrats aus der Abtrennung des Mononatriumsalzes des 2,4,6-Trimercapto-s-triazins (TMT-Na₁) auf einen pH-Wert von 1,5 bis 2,5 eingestellt wird.

5. Verfahren gemäß Anspruch 4,
**dadurch gekennzeichnet,**
**dass** das ausfallende 2,4,6-Trimercapto-s-triazin (TMT-H₃) mittels eines Trennverfahrens abgetrennt wird und das erhaltene 2,4,6-Trimercapto-s-triazin (TMT-H₃) der Mutterlauge vor dem Ausfällen des Mononatriumsalzes des 2,4,6-Trimercapto-s-triazins (TMT-Na₁) zugeführt wird.

6. Verfahren gemäß Anspruch 3,
**dadurch gekennzeichnet,**
**dass** das Mononatriumsalz des 2,4,6-Trimercapto-s-triazins (TMT-Na₁) mittels Natronlauge bei einem pH-Wert von 10 bis 11 und einer Temperatur von 20 bis 50°C in Lösung gebracht wird, wobei sich das Dinatriumsalz des 2,4,6-Trimercapto-s-triazins (TMT-Na₂) bildet.

7. Verfahren gemäß Anspruch 6,
**dadurch gekennzeichnet,**
**dass** die wässrige Lösung des Dinatriumsalzes des 2,4,6-Trimercapto-s-triazins (TMT-Na₂) in den Reaktor zurückgeführt wird.

8. Verfahren gemäß Anspruch 1,
**dadurch gekennzeichnet,**
**dass** der Mutterlauge vor dem Ausfällen des Mononatriumsalzes des 2,4,6-Trimercapto-s-triazins (TMT-Na₁) ein Unterdruck von < 1000 mbar angelegt wird.

9. Verfahren gemäß Anspruch 8,
**dadurch gekennzeichnet,**
**dass** bei einer Temperatur von 10 °C bis 55 °C die Mutterlauge entgast wird.

10. Verfahren gemäß Anspruch 8 oder 9,
**dadurch gekennzeichnet,**
**dass** die entweichenden Gase durch ein oder mehrere Gaswäscher mit Natronlauge geleitet werden.

11. Verfahren gemäß Anspruch 10,
**dadurch gekennzeichnet,**
**dass** die wässrige Lösung an NaSH, Na₂S und Natronlauge in den Reaktor zurückgeführt wird.

## Claims

1. Process for preparing the trisodium salt of 2,4,6-trimercapto-s-triazine (TMT-Na₃), where cyanuric chloride is reacted in an aqueous medium with NaSH, Na₂S or a mixture of these two compounds at a temperature of from 20 to 70°C and a pH of from 7 to 11 and the trisodium salt of 2,4,6-trimercapto-s-triazine (TMT-Na₃) is subsequently precipitated and separated off at a pH of ≥ 12 and a temperature of from 0 to 20°C, **characterized in that** the mother liquor after the trisodium salt of 2,4,6-trimercapto-s-triazine (TMT-Na₃) has been separated off is set to a pH of from 6 to 8, resulting in the monosodium salt of 2,4,6-trimercapto-s-triazine (TMT-Na₁) precipitating.

2. Process according to Claim 1, **characterized in that** the mother liquor is set to a temperature of from 10 to 45°C.

3. Process according to Claim 1 or 2, **characterized in that** the precipitated monosodium salt of 2,4,6-trimercapto-s-triazine (TMT-Na₁) is separated off from the mother liquor by means of a mechanical separation process.

4. Process according to Claim 3, **characterized in that** the pH of the filtrate from the isolation of the monosodium salt of 2,4,6-trimercapto-s-triazine (TMT-Na₁) is set to a pH of from 1.5 to 2.5.

5. Process according to Claim 4, **characterized in that** the 2,4,6-trimercapto-s-triazine (TMT-H₃) which precipitates is separated off by means of a separation process and the 2,4,6-trimercapto-s-triazine (TMT-H₃) obtained is added to the mother liquor before precipitation of the monosodium salt of 2,4,6-trimercapto-s-triazine (TMT-Na₁).

6. Process according to Claim 3, **characterized in that** the monosodium salt of 2,4,6-trimercapto-s-triazine (TMT-Na₁) is brought into solution by means of sodium hydroxide at a pH of from 10 to 11 and a temperature of from 20 to 50°C, forming the disodium salt of 2,4,6-trimercapto-s-triazine (TMT-Na₂).

7. Process according to Claim 6, **characterized in that** the aqueous solution of the disodium salt of 2,4,6-trimercapto-s-triazine (TMT-Na₂) is recirculated to the reactor.

8. Process according to Claim 1, **characterized in that** a subatmospheric pressure of < 1000 mbar is applied to the mother liquor before precipitation of the monosodium salt of 2,4,6-trimercapto-s-triazine (TMT-Na₁).

9. Process according to Claim 8, **characterized in that** the mother liquor is degassed at a temperature of from 10°C to 55°C.

10. Process according to Claim 8 or 9, **characterized in that** the gases given off are passed through one or more gas scrubbers containing sodium hydroxide.

11. Process according to Claim 10, **characterized in that** the aqueous solution of NaSH, Na₂S and sodium hydroxide is recirculated to the reactor.

## Revendications

1. Procédé de préparation du sel trisodique de 2,4,6-trimercapto-s-triazine (TMT-Na₃), selon lequel du chlorure de cyanure est mis en réaction dans un milieu aqueux à une température de 20 à 70 °C avec NaSH, Na₂S ou un mélange de ces deux composés à un pH de 7 à 11, puis le sel trisodique de 2,4,6-trimercapto-s-triazine (TMT-Na₃) est précipité à un pH ≥ 12 et à une température de 0 à 20 °C et séparé,
**caractérisé en ce que**
la liqueur mère est ajustée à un pH de 6 à 8 après l'étape de séparation du sel trisodique de 2,4,6-trimercapto-s-triazine (TMT-Na₃), le sel monosodique de 2,4,6-trimercapto-s-triazine (TMT-Na₁) précipitant.

2. Procédé selon la revendication 1, **caractérisé en ce que** la liqueur mère est ajustée à une température de 10 à 45 °C.

3. Procédé selon la revendication 1 ou 2, **caractérisé en ce que** le sel monosodique de 2,4,6-trimercapto-s-triazine (TMT-Na₁) précipité est séparé de la liqueur mère par un procédé de séparation mécanique.

4. Procédé selon la revendication 3, **caractérisé en ce que** le pH du filtrat issu de la séparation du sel monosodique de 2,4,6-trimercapto-s-triazine (TMT-Na₁) est ajusté à un pH de 1,5 à 2,5.

5. Procédé selon la revendication 4, **caractérisé en ce que** la 2,4,6-trimercapto-s-triazine (TMT-H₃) précipitée est séparée par un procédé de séparation et la 2,4,6-trimercapto-s-triazine (TMT-H₃) obtenue est introduite dans la liqueur mère avant la précipitation du sel monosodique de 2,4,6-trimercapto-s-triazine (TMT-Na₁).

6. Procédé selon la revendication 3, **caractérisé en ce que** le sel monosodique de 2,4,6-trimercapto-s-triazine (TMT-Na₁) est mis en solution par de la soude caustique à un pH de 10 à 11 et à une température de 20 à 50 °C, le sel disodique de 2,4,6-trimercapto-s-triazine (TMT-Na₂) se formant.

7. Procédé selon la revendication 6, **caractérisé en ce que** la solution aqueuse du sel disodique de 2,4,6-trimercapto-s-triazine (TMT-Na₂) est recyclée dans le réacteur.

8. Procédé selon la revendication 1, **caractérisé en ce qu'**une sous-pression < 1 000 mbar est appliquée à la liqueur mère avant la précipitation du sel monosodique de 2,4,6-trimercapto-s-triazine (TMT-Na₁).

9. Procédé selon la revendication 8, **caractérisé en ce que** la liqueur mère est dégazée à une température de 10 °C à 55 °C.

10. Procédé selon la revendication 8 ou 9, **caractérisé en ce que** les gaz libérés sont conduits dans un ou plusieurs épurateurs de gaz contenant de la soude caustique.

11. Procédé selon la revendication 10, **caractérisé en ce que** la solution aqueuse de NaSH, Na₂S et de soude caustique est recyclée dans le réacteur.
